(19) Europäisches Patentamt
European Patent Office
Office européen des brevets

(11) **EP 4 130 739 A1**

(12) **EUROPEAN PATENT APPLICATION**
published in accordance with Art. 153(4) EPC

(43) Date of publication:
**08.02.2023 Bulletin 2023/06**

(21) Application number: **21775201.3**

(22) Date of filing: **24.03.2021**

(51) International Patent Classification (IPC):
**G01N 33/483** (1985.01)  **G01N 33/52** (1980.01)
**G01N 21/64** (1980.01)  **G01N 21/78** (1980.01)

(52) Cooperative Patent Classification (CPC):
**G01N 21/64; G01N 21/78; G01N 33/483;**
**G01N 33/52**

(86) International application number:
**PCT/JP2021/012167**

(87) International publication number:
**WO 2021/193700 (30.09.2021 Gazette 2021/39)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **25.03.2020 JP 2020054061**

(71) Applicant: **FUJIFILM Corporation**
**Tokyo 106-8620 (JP)**

(72) Inventors:
• **FUJITA Masaharu**
**Ashigarakami-gun, Kanagawa 258-8577 (JP)**
• **YAMAMOTO Yusuke**
**Ashigarakami-gun, Kanagawa 258-8577 (JP)**
• **KASAHARA Toshihiko**
**Ashigarakami-gun, Kanagawa 258-8577 (JP)**

(74) Representative: **HGF**
**HGF Limited**
**1 City Walk**
**Leeds LS11 9DX (GB)**

(54) **METHOD FOR MEASURING RESPIRATORY SENSITIZATION AND RESPIRATORY SENSITIZATION MEASURING REAGENT**

(57)    An object of the present invention is to provide a method for measuring respiratory sensitization and a respiratory sensitization measuring reagent that can be used to evaluate a test substance for respiratory sensitization without using an animal. According to the present invention, there is provided a method for measuring respiratory sensitization, including reacting a N-(arylalkylcarbonyl)cysteine with a test substance; reacting an α-N-(arylalkylcarbonyl)lysine with the test substance; detecting the amount of each of the above compounds or a product thereof after the reaction by optical measurement; and determining respiratory sensitization from the ratio of the reactivity of the α-N-(arylalkylcarbonyl)lysine with the test substance to the reactivity of the N-(arylalkylcarbonyl)cysteine with the test substance or from the reactivity of the α-N-(arylalkylcarbonyl)lysine with the test substance.

EP 4 130 739 A1

**Description**

Technical Field

The present invention relates to a method for measuring respiratory sensitization and a respiratory sensitization measuring reagent.

Background Art

Sensitization (allergy) refers to an excessive immune response to a specific antigen. An immune response is a physiological function essential for living organisms that works to eliminate foreign substances (antigens). However, an immune response is considered to be one of the important toxicities because, for example, once it develops, management for avoiding exposure to the antigen is required over a long period of time.

Allergic reactions are classified into four types: Types I to IV Of these types, skin sensitization, which is a delayed allergy, is classified as a Type IV allergy. On the other hand, many known allergic diseases are Type I allergies, which are classified as immediate allergies, including respiratory sensitization such as bronchial asthma and allergic rhinitis.

Not less than 300 respiratory sensitizers cause occupational asthma, and the World Health Survey (WHS) estimates that over 300,000,000 people suffer from asthma. Known chemical substances that cause respiratory sensitization include acid anhydrides, isocyanates, reactive dyes, and chloroplatinates, which are used in resins, coatings, foams, and other various industries.

There is no in vivo test method that is well-established and widely accepted as a respiratory sensitization test method. On the other hand, in vitro and in chemico test methods are known as attempts to detect respiratory sensitizers by a simple test method. As an in vitro test method, a test method for evaluating a respiratory sensitizer by analyzing the expression level of a specific gene in cultured dendritic cells has been proposed (Non-Patent Document 1). However, there are only a limited number of respiratory sensitizers that have been evaluated, namely, three respiratory sensitizers, and whether a wide range of respiratory sensitizers can be evaluated is considered to be a future issue. In addition, among many in vitro test methods developed as alternative skin sensitization test methods, there is no known method capable of predicting respiratory sensitizers while distinguishing them from skin sensitizers.

As an in chemico test method, there is a report on the evaluation of respiratory sensitizers by the direct peptide reactivity assay (DPRA) (Non-Patent Document 2). According to the report, in this method, the reactivity (depletion) of cysteine peptide and lysine peptide with 17 respiratory sensitizers was calculated from high-performance liquid chromatography (HPLC) measurement, and the average ratio of the depletion of lysine peptide to the depletion of cysteine peptide was 4.6. However, there is no defined criterion for determining respiratory sensitizers; therefore, this method cannot be used as a method for predicting respiratory sensitizers.

On the other hand, Patent Document 1 describes a skin sensitization measuring reagent using a N-(arylalkylcarbonyl)cysteine, and Patent Document 2 describes a skin sensitization measuring reagent using an $\alpha$-N-(arylalkylcarbonyl)lysine. The assay using the skin sensitization measuring reagents described in Patent Document 1 and Patent Document 2 is also known as the amino acid derivative reactivity assay (ADRA).

Prior Art Documents

Non-Patent Documents

Non-Patent Document 1: Mizoguchi I, Ohashi M, Chiba Y, Hasegawa H, Xu M, Owaki T, and Yoshimoto T. 2017. Prediction of chemical respiratory and contact sensitizers by OX40L expression in dendric cells using a novel 3D coculture system. Frontiers in Immunology, 8, 929.

Non-Patent Document 2: Lalko JF, Kimber I, Gerberick GF, Foertsch LM, Api AM, Dearman RJ. (2012). The direct peptide reactivity assay: selectivity of chemical respiratory allergens. Toxicological Sciences, 129(2), 421-431.

Patent Documents

Patent Document 1: JP2001-59102A
Patent Document 2: JP2014-37995A

**SUMMARY OF THE INVENTION**

Since animal testing has many problems such as complicated procedures, long testing periods, and high testing costs, and there is also a social need from the viewpoint of animal welfare, efforts have been made to develop alternative methods to animal testing that do not use animals. In particular, there is an urgent need for the development of an alternative method to respiratory sensitization tests that show serious symptoms. Although in vitro tests using cultured cells and in chemico tests based on chemical reactions have been studied, both of them are still in the early stage of research, and no satisfactory prediction results have been obtained.

Although respiratory sensitizers pose a risk to human health, no test method capable of identifying respiratory sensitizers has so far been established. To date, only a few in vitro models for studying respiratory sensitization have been developed, and these models have yet to be verified and require a further study.

In the DPRA described in Non-Patent Document 2, two reagents, namely, cysteine peptide and lysine peptide, are used and separately reacted with a test substance. These reaction solutions are separately subjected to high-performance liquid chromatography (HPLC). The depletion of each peptide is calculated from the amount of unreacted peptide, and the average thereof (average depletion) is calculated. Since many respiratory sensitizers are known to have high reactivity with lysine, the predictability of respiratory sensitizers by DPRA has been studied. However, there are cases where respiratory sensitizers cannot be correctly evaluated by evaluating their reactivity with lysine peptide. In addition, there is no disclosure of a definite criterion based on which respiratory sensitizers can be determined in Non-Patent Document 2.

An object of the present invention is to provide a method for measuring respiratory sensitization and a respiratory sensitization measuring reagent that can be used to evaluate a test substance for respiratory sensitization without using an animal.

As a result of intensive studies to achieve the above object, the present inventors have found that respiratory sensitization can be measured by using a N-(arylalkylcarbonyl)cysteine and an α-N-(arylalkylcarbonyl)lysine. The present invention has been completed based on these findings.

That is, according to the present invention, the following inventions are provided:

<1> A method for measuring respiratory sensitization, including:

reacting a N-(arylalkylcarbonyl)cysteine with at least one test substance;
reacting an α-N-(arylalkylcarbonyl)lysine with the at least one test substance;
detecting the amount of the N-(arylalkylcarbonyl)cysteine or a product thereof after the reaction and the amount of the α-N-(arylalkylcarbonyl)lysine or a product thereof after the reaction by optical measurement using an ultraviolet detector or a fluorescence detector; and
determining respiratory sensitization from the ratio of the reactivity of the α-N-(arylalkylcarbonyl)lysine with the test substance to the reactivity of the N-(arylalkylcarbonyl)cysteine with the test substance or from the reactivity of the α-N-(arylalkylcarbonyl)lysine with the test substance.

<2> The method for measuring respiratory sensitization according to <1>, wherein the N-(arylalkylcarbonyl)cysteine and the α-N-(arylalkylcarbonyl)lysine are compounds that exhibit absorption in a wavelength range of 200 to 700 nm and that have a molar absorption coefficient of 10 L/mol cm or more and 500,000 L/mol cm or less at a maximal absorption wavelength, or are compounds that emit fluorescence at 200 to 400 nm at an excitation wavelength of 200 to 350 nm.

<3> The method for measuring respiratory sensitization according to <1> or <2>, wherein the N-(arylalkylcarbonyl)cysteine is N-(2-phenylacetyl)cysteine or N-[2-(naphthalen-1-yl)acetyl]cysteine.

<4> The method for measuring respiratory sensitization according to any one of <1> to <3>, wherein the α-N-(arylalkylcarbonyl)lysine is α-N-(2-phenylacetyl)lysine or α-N-[2-(naphthalen-1-yl)acetyl]lysine.

<5> The method for measuring respiratory sensitization according to any one of <1> to <4>, wherein the N-(arylalkylcarbonyl)cysteine and the α-N-(arylalkylcarbonyl)lysine are each prepared at a concentration of 0.1 μmol/L to 100 μmol/L.

<6> The method for measuring respiratory sensitization according to any one of <1> to <5>, wherein

reacting the N-(arylalkylcarbonyl)cysteine with the at least one test substance includes reacting a solution of the N-(arylalkylcarbonyl)cysteine with a solution of the test substance in water, an organic solvent, or a mixture thereof at a concentration of 0.1 mmol/L to 10 mmol/L if the molecular weight of the test substance is known or at a concentration of 0.05 mg/mL to 10 mg/mL if the molecular weight of the test substance is unknown, and
reacting the α-N-(arylalkylcarbonyl)lysine with the at least one test substance includes reacting a solution of the α-N-(arylalkylcarbonyl)lysine with a solution of the test substance in water, an organic solvent, or a mixture thereof at a concentration of 0.1 mmol/L to 10 mmol/L if the molecular weight of the test substance is known or at a concentration of 0.05 mg/mL to 10 mg/mL if the molecular weight of the test substance is unknown.

<7> The method for measuring respiratory sensitization according to any one of <1> to <6>, further including chromatographing a product obtained by reacting the N-(arylalkylcarbonyl)cysteine with the at least one test substance and a product obtained by reacting the α-N-(arylalkylcarbonyl)lysine with the at least one test substance.

<8> The method for measuring respiratory sensitization according to any one of <1> to <7>, wherein the detection wavelength in the optical measurement using an ultraviolet detector is 200 to 700 nm.

<9> The method for measuring respiratory sensitization according to any one of <1> to <8>, wherein, in the optical measurement using a fluorescence detector, the excitation wavelength is 200 to 350 nm, and the fluorescence wavelength is 200 to 400 nm.

<10> The method for measuring respiratory sensitization according to any one of <1> to <9>, wherein the at least one test substance is at least one of a perfume, an essential oil, a polymer compound, a medicine, an agricultural chemical, a food, a chemical product, or a plant extract made of a naturally derived component.

<11> The method for measuring respiratory sensitization according to any one of <1> to <10>, wherein the depletion of the N-(arylalkylcarbonyl)cysteine and the depletion of the α-N-(arylalkylcarbonyl)lysine are calculated from the average peak area of the N-(arylalkylcarbonyl)cysteine and the average peak area of the α-N-(arylalkylcarbonyl)lysine in the optical measurement using an ultraviolet detector or a fluorescence detector by the following equations:

depletion (% depletion) of N-(arylalkylcarbonyl)cysteine = [1 - (average peak area of unreacted N-(arylalkylcarbonyl)cysteine after reaction/average peak area of standard N-(arylalkylcarbonyl)cysteine)] × 100

depletion (% depletion) of α-N-(arylalkylcarbonyl)lysine = [1 - (average peak area of unreacted α-N-(arylalkylcarbonyl)lysine after reaction/average peak area of standard α-N-(arylalkylcarbonyl)lysine)] × 100

<12> The method for measuring respiratory sensitization according to <11>, wherein

the reaction of the N-(arylalkylcarbonyl)cysteine with the at least one test substance and the reaction of the α-N-(arylalkylcarbonyl)lysine with the at least one test substance are separately carried out, and
the test substance is determined to be a respiratory sensitizer if
[C] ≥ 4.9% and [B]/[A] ≥ 0.9 are satisfied, or
[B] ≥ 20% is satisfied,

where [A] represents the depletion of the N-(arylalkylcarbonyl)cysteine, [B] represents the depletion of the α-N-(arylalkylcarbonyl)lysine, and [C] represents the average of [A] and [B].

<13> The method for measuring respiratory sensitization according to <11>, wherein

the reaction of the N-(arylalkylcarbonyl)cysteine with the at least one test substance and the reaction of the α-N-(arylalkylcarbonyl)lysine with the at least one test substance are carried out using a mixed solution with a pH of 8.0 including the N-(arylalkylcarbonyl)cysteine and the α-N-(arylalkylcarbonyl)lysine, and
the test substance is determined to be a respiratory sensitizer if
[C] ≥ 4.9% and [B]/[A] ≥ 0.9 are satisfied, or
[B] ≥ 10% is satisfied,

where [A] represents the depletion of the N-(arylalkylcarbonyl)cysteine, [B] represents the depletion of the α-N-(arylalkylcarbonyl)lysine, and [C] represents the average of [A] and [B].

<14> The method for measuring respiratory sensitization according to <13>, wherein

after the test substance is determined not to be a respiratory sensitizer by the determination in <13>,
the reaction of the N-(arylalkylcarbonyl)cysteine with the at least one test substance and the reaction of the α-N-(arylalkylcarbonyl)lysine with the at least one test substance are carried out using a mixed solution with a pH of 10.2 including the N-(arylalkylcarbonyl)cysteine and the α-N-(arylalkylcarbonyl)lysine, and
the test substance is determined to be a respiratory sensitizer if [B] ≥ 20% is satisfied.

<15> The method for measuring respiratory sensitization according to any one of <1> to <14>, wherein a diisocyanate is determined to be a respiratory sensitizer by the determination.

<16> A respiratory sensitization measuring reagent for measuring respiratory sensitization of at least one test substance by optical measurement using an ultraviolet detector, the respiratory sensitization measuring reagent including a N-(arylalkylcarbonyl)cysteine or an α-N-(arylalkylcarbonyl)lysine as a main measuring agent.

According to the present invention, a test substance can be evaluated for respiratory sensitization without using an animal.

## DESCRIPTION OF THE PREFERRED EMBODIMENTS

In the present specification, "to" is used to mean that numerical values recited before and after "to" are included as lower and upper limits.

The present invention relates to a method for measuring respiratory sensitization, including:

reacting a N-(arylalkylcarbonyl)cysteine with at least one test substance;
reacting an α-N-(arylalkylcarbonyl)lysine with the at least one test substance;
detecting the amount of the N-(arylalkylcarbonyl)cysteine or a product thereof after the reaction and the amount of the α-N-(arylalkylcarbonyl)lysine or a product thereof after the reaction by optical measurement using an ultraviolet detector or a fluorescence detector; and
determining respiratory sensitization from the ratio of the reactivity of the α-N-(arylalkylcarbonyl)lysine with the test substance to the reactivity of the N-(arylalkylcarbonyl)cysteine with the test substance or from the reactivity of the α-N-(arylalkylcarbonyl)lysine with the test substance.

The present invention further relates to a respiratory sensitization measuring reagent for measuring respiratory sensitization of at least one test substance by optical measurement using an ultraviolet detector, the respiratory sensitization measuring reagent including a N-(arylalkylcarbonyl)cysteine or an α-N-(arylalkylcarbonyl)lysine as a main measuring agent.

As with skin sensitization, at the initial stage of the development mechanism of respiratory sensitization, a test substance penetrates into a living body and then covalently bonds to biological proteins. Here, it is believed that the amino acids in the biological proteins involved in this covalent bonding are the SH group of cysteine and the $NH_2$ group of lysine, of which the $NH_2$ group of lysine is known to be mainly involved. Accordingly, ADRA has been developed as a test method for determining the reactivity of a N-(arylalkylcarbonyl)cysteine and an α-N-(arylalkylcarbonyl)lysine with a test substance. The N-(arylalkylcarbonyl)cysteine and the α-N-(arylalkylcarbonyl)lysine are chemically synthesized by introducing an aryl group ring having a high molar absorption coefficient and fluorescence in the UV region into the N-termini of cysteine and lysine. These two nucleophilic reagents are reacted with the test substance, and the unreacted nucleophilic reagents are quantified. In the present invention, a respiratory sensitizer can be evaluated by using, as an index, the ratio of the reactivity of the α-N-(arylalkylcarbonyl)lysine with the test substance to the reactivity of the N-(arylalkylcarbonyl)cysteine with the test substance, or the reactivity of the α-N-(arylalkylcarbonyl)lysine with the test substance.

The present invention is advantageous in that the N-(arylalkylcarbonyl)cysteine or the α-N-(arylalkylcarbonyl)lysine can be used as a reagent to measure respiratory sensitization without using an animal. In addition, in the present invention, the N-(arylalkylcarbonyl)cysteine and the α-N-(arylalkylcarbonyl)lysine can be detected by fluorescence detection. This can be used to quantify the reagents while completely distinguishing them from the test substance.

By "measurement of respiratory sensitization" in the present invention, it is meant to include an assay for measurement of respiratory sensitization, and it is also meant to include determination of the presence or absence of respiratory sensitization based on a certain criterion and quantitative measurement of respiratory sensitization.

In the present invention, the N-(arylalkylcarbonyl)cysteine or the α-N-(arylalkylcarbonyl)lysine is used. These two reagents are reacted with the test substance, and the amount of the N-(arylalkylcarbonyl)cysteine or a product thereof after the reaction and the amount of the α-N-(arylalkylcarbonyl)lysine or a product thereof after the reaction are detected by optical measurement using an ultraviolet detector or a fluorescence detector. Thereafter, respiratory sensitization is determined from the ratio of the reactivity of the α-N-(arylalkylcarbonyl)lysine with the test substance to the reactivity of the N-(arylalkylcarbonyl)cysteine with the test substance or from the reactivity of the α-N-(arylalkylcarbonyl)lysine with the test substance.

In the present invention, the N-(arylalkylcarbonyl)cysteine or the α-N-(arylalkylcarbonyl)lysine is used.

The N-(arylalkylcarbonyl)cysteine is preferably a compound that exhibits absorption in a wavelength range of 190 to 2,500 nm, more preferably a compound that exhibits absorption in a wavelength range of 200 to 700 nm, as is or in solution form. A compound having maximal absorption in the above wavelength range is further preferred. The N-(arylalkylcarbonyl)cysteine is also preferably a compound having a molar absorption coefficient of 10 L/mol cm or more and 500,000 L/mol cm or less at the maximal absorption wavelength, more preferably a compound having a molar absorption coefficient of 10 L/mol·cm or more and 2,000 L/mol·cm or less at the maximal absorption wavelength, and even more preferably a compound having a molar absorption coefficient of 100 L/mol·cm or more and 2,000 L/mol·cm or less at the maximal absorption wavelength.

The N-(arylalkylcarbonyl)cysteine is also preferably a compound that emits fluorescence at 200 to 400 nm at an excitation wavelength of 200 to 350 nm.

The aryl group of the N-(arylalkylcarbonyl)cysteine may have about 6 to 16 carbon atoms. In addition, the alkylcarbonyl group may have about 2 to 11 carbon atoms. The alkyl group attached to the carbonyl group may be linear, branched, or cyclic, and examples thereof include a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a hexyl group, a heptyl group, an octyl group, a nonyl group, a decyl group, a 2-ethylhexyl group, and a cyclopropyl group. Specific examples of such compounds include N-(2-phenylacetyl)cysteine (hereinafter also referred to as PAC) and N-[2-(naphthalen-1-yl)acetyl]cysteine (hereinafter also referred to as NAC).

The N-(arylalkylcarbonyl)cysteine can be produced by a known method. For example, N-(2-phenylacetyl)cysteine can be synthesized by the method described in paragraphs 0015 to 0017 of JP2011-59102A.

N-[2-(Naphthalen-1-yl)acetyl]cysteine can be synthesized by the following method.

In 270 mL of toluene, 50 g of 1-naphthylacetic acid is dissolved, and 95. 8 g of thionyl chloride is added dropwise at 20°C, with N,N-dimethylformamide (DMF) being added dropwise. The reaction is carried out at 60°C for 2 hours, followed by cooling. After 200 mL of toluene is added, the mixture is dried under reduced pressure to obtain 1-naphthylacetyl chloride (56 g).

To an aqueous solution of 14 g of sodium hydroxide in 350 mL of water, 20 g of L-cystine is added and dissolved. The solution is cooled in an ice water bath, and 8.76 g of 1-naphthylacetyl chloride is added dropwise. The reaction mixture is stirred at 20°C for 2 hours. After cooling, 16.7 mL of concentrated hydrochloric acid is added. About 700 mL of ethyl acetate is added, and a crystal is filtered off and is dried under reduced pressure to obtain N,N'-bis(1-naphthylacetyl)cystine (39.2 g).

To a mixture of 20 g of N,N'-bis(1-naphthylacetyl)cystine, 20 g of zinc powder, and 500 mL of methanol, 120 mL of trifluoroacetic acid is added dropwise under nitrogen purge over 2 hours. After the reaction mixture is stirred for 2 hours, extraction is carried out with 500 mL of ethyl acetate, and the organic phase is dried over magnesium sulfate and is then filtered and concentrated. The resulting residue is recrystallized from ethyl acetate and is dried to obtain N-[2-(naphthalen-1-yl)acetyl]cysteine (5.2 g).

N-[2-(Naphthalen-1-yl)acetyl]cysteine emits fluorescence at 200 to 400 nm, exhibits maximal absorption at 281 nm, and has a molar absorption coefficient of about 7,000 (L/mol·cm) and a maximum fluorescence wavelength of 335 nm.

The α-N-(arylalkylcarbonyl)lysine, having an amino group, is also reactive with substances having low reactivity with the N-(arylalkylcarbonyl)cysteine and can be analyzed using a general-purpose simple analyzer. The α-N-(arylalkylcarbonyl)lysine also has sufficient degrees of solubility and stability in a reaction solution containing a high proportion of organic solvent for dissolution of hydrophobic chemical substances.

The α-N-(arylalkylcarbonyl)lysine is preferably a compound that exhibits absorption in a wavelength range of 190 to 2,500 nm, more preferably a compound that exhibits absorption in a wavelength range of 200 to 700 nm, as is or in solution form. A compound having maximal absorption in the above wavelength range is further preferred. The α-N-(arylalkylcarbonyl)lysine is preferably a compound having a molar absorption coefficient of 10 L/mol·cm or more and 500,000 L/mol·cm or less at the maximal absorption wavelength, more preferably a compound having a molar absorption coefficient of 10 L/mol·cm or more and 2,000 L/mol·cm or less at the maximal absorption wavelength, and even more preferably a compound having a molar absorption coefficient of 100 L/mol·cm or more and 2,000 L/mol·cm or less at the maximal absorption wavelength.

The α-N-(arylalkylcarbonyl)lysine is also preferably a compound that emits fluorescence at 200 to 400 nm at an excitation wavelength of 200 to 350 nm.

The molar absorption coefficient ($\varepsilon$) is given by the following equation:

$$\varepsilon = D/(c \cdot d)$$

where D represents the absorbance of the solution, c represents the molar concentration (mol/L) of the solute, and d represents the thickness (optical path length) (cm) of the solution layer. The molar absorption coefficient can be determined by measuring the absorption spectrum or absorbance using a commercially available spectrophotometer.

The aryl group of the α-N-(arylalkylcarbonyl)lysine may have about 6 to 16 carbon atoms. Examples of aryl groups include a benzene ring and a naphthalene ring. In addition, the alkylcarbonyl group may have about 2 to 11 carbon atoms. The alkyl group attached to the carbonyl group may be linear, branched, or cyclic, and examples thereof include a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a hexyl group, a heptyl group, an octyl group, a nonyl group, a decyl group, a 2-ethylhexyl group, and a cyclopropyl group. Specific examples of α-N-(arylalkylcarbonyl)lysines include α-N-(2-phenylacetyl)lysine (hereinafter also referred to as PAL) and α-N-[2-(naphthalen-1-yl)acetyl]lysine (hereinafter also referred to as NAL).

The α-N-(arylalkylcarbonyl)lysine can be produced by a known method. For example, PAL and NAL can be synthesized by the method described in paragraphs 0025 to 0031 of JP2014-37995A.

α-N-(2-Phenylacetyl)lysine emits fluorescence at 200 to 400 nm and has a molar absorption coefficient of about 200 L/mol·cm at the maximal absorption wavelength (around 255 nm).

α-N-[2-(Naphthalen-1-yl)acetyl]lysine emits fluorescence at 200 to 400 nm, has a molar absorption coefficient of about 400 L/mol cm at the maximal absorption wavelength (around 280 nm), and has a maximum fluorescence wavelength of 332 nm.

The respiratory sensitization measuring reagent according to the present invention may be composed only of the above N-(arylalkylcarbonyl)cysteine or α-N-(arylalkylcarbonyl)lysine or may include one or more additives in addition to the above compound serving as the main measuring agent. Examples of additives include pH adjusters, stabilizers, chelating agents, and reducing agents. The respiratory sensitization measuring reagent according to the present invention may also be a solution of the above main measuring agent and optionally the above additives in water, an aqueous buffer solution, an organic solvent, or a mixture thereof. The respiratory sensitization measuring reagent according to the present invention may be provided in solution form, liquid form, or solid form (e.g., in powder, granular, freeze-dried, or tablet form).

For example, the respiratory sensitization measuring reagent according to the present invention may be used in the form of a solution in water or an aqueous buffer solution including an organic acid salt such as ammonium acetate or an inorganic salt such as a phosphate, or in a mixture thereof with an organic solvent, for example, at a concentration of about 0.01 μmol/L to 1 mol/L, typically about 0.1 μmol/L to 500 mmol/L, preferably about 0.1 μmol/L to 100 μmol/L.

In the present invention, when the N-(arylalkylcarbonyl)cysteine is reacted with a test substance, and when the α-N-(arylalkylcarbonyl)lysine is reacted with a test substance, a single test substance may be reacted, or a mixture containing two or more test substances may be reacted. "Mixture containing two or more test substances" refers to a mixture including two or more test substances as components corresponding to main components, and does not refer to a mixture including a single test substance and many impurities.

A specific example of a single test substance or a mixture including two or more test substances may be, but is not particularly limited to, at least one of a perfume, an essential oil, a polymer compound, a medicine, an agricultural chemical, a food, a chemical product, or a plant extract made of a naturally derived component.

If the molar concentration of the test substance can be adjusted, the test substance may be dissolved in, for example, water, an organic solvent compatible with water (e.g., methanol, ethanol, acetonitrile, acetone, or N,N-dimethyl sulfoxide (DMSO)), or a mixture thereof (a mixture of water and an organic solvent, or a mixture of two or more organic solvents).

If the molecular weight of the test substance is known, the test substance is preferably dissolved at a concentration of 0.1 mmol/L to 100 mmol/L, more preferably dissolved at a concentration of 0.5 mmol/L to 50 mmol/L, and even more preferably dissolved at a concentration of 1 mmol/L to 10 mmol/L.

Each of the N-(arylalkylcarbonyl)cysteine and the α-N-(arylalkylcarbonyl)lysine, which serve as the main measuring agent of the respiratory sensitization measuring reagent according to the present invention, may then be mixed and reacted with the test substance solution such that the molar ratio of each of the above compounds to the test substance is, for example, 1:100 to 20:1, or 1:100 to 10:1.

Even if the molar concentration of the test substance cannot be adjusted, the test substance may be dissolved in, for example, water, an organic solvent compatible with water (e.g., methanol, ethanol, acetonitrile, acetone, or N,N-dimethyl sulfoxide (DMSO)), or a mixture thereof (a mixture of water and an organic solvent, or a mixture of two or more organic solvents).

If the molecular weight of the test substance is unknown, the test substance is preferably dissolved at a concentration of 0.01 mg/mL to 10 mg/mL, more preferably dissolved at a concentration of 0.5 mg/mL to 5 mg/mL, and even more preferably dissolved at a concentration of 0.1 mg/mL to 1 mg/mL.

About 0.1 to about 100 μg/mL, typically 1 to 10 μg/mL, solutions of the N-(arylalkylcarbonyl)cysteine and the α-N-(arylalkylcarbonyl)lysine, which serve as the main measuring agent of the respiratory sensitization measuring reagent according to the present invention, may then be added to the test substance solution in equal amounts. However, appropriate adjustment may be made as long as the concentration and the amount added are as described above. For example, the concentration of the measuring reagent may be doubled while the amount added is halved.

The concentration of each of the N-(arylalkylcarbonyl)cysteine and the α-N-(arylalkylcarbonyl)lysine in the reaction solutions for the reaction of the N-(arylalkylcarbonyl)cysteine and the α-N-(arylalkylcarbonyl)lysine with the test substance is preferably 0.05 μmol/L or more and 400 μmol/L or less, more preferably 0.1 μmol/L or more and 100 μmol/L or less, and even more preferably 1.0 μmol/L or more and 10 μmol/L or less.

The reaction can be carried out by stirring a solution including the N-(arylalkylcarbonyl)cysteine and/or the α-N-(arylalkylcarbonyl)lysine and the test substance, or allowing the solution to stand, in a temperature range of, for example, about 4°C to about 60°C, preferably about 10°C to about 50°C, more preferably about 15°C to about 40°C, typically for about 1 minute to about 2 days, preferably for 1 hour to 2 days, more preferably for 8 hours to 36 hours, optionally while warming the solution. The reaction between the N-(arylalkylcarbonyl)cysteine and at least one test substance and the reaction between the α-N-(arylalkylcarbonyl)lysine and the at least one test substance may be carried out in separate reaction solutions or may be carried out in the same reaction solution.

Whereas the reactivity (covalent bonding ability) of the nucleophilic reagent (i.e., the N-(arylalkylcarbonyl)cysteine or the α-N-(arylalkylcarbonyl)lysine) with the test substance can be estimated by quantifying the unreacted nucleophilic reagent, the most direct and correct evaluation is to detect and quantify the product of the reaction of the nucleophilic reagent with the test substance (reaction product). A mixture can be subjected to quantification, for example, by high-performance liquid chromatography (HPLC)-fluorescence detection if its components are known and the reaction products of the components with the nucleophilic reagent are available.

Although the method for analyzing the nucleophilic reagent or the reaction product is not particularly limited, the method preferably includes chromatographing the product obtained in the step of reacting the nucleophilic reagent with the test substance. For example, a method can be used in which the compound produced by the above reaction, the nucleophilic reagent, and the test substance are separated and analyzed by high-performance liquid chromatography (HPLC), gas chromatography (GC), thin-layer chromatography (TLC), or the like. Examples of chromatographic techniques that can be used for HPLC, GC, or TLC above include reverse-phase techniques, normal-phase techniques, and ion-exchange techniques. Examples of commercially available columns and TLC plates that can be used for such chromatographic techniques include LC columns such as CAPCELL CORE C18 (manufactured by Osaka Soda Co., Ltd.), CAPCELL-PAK (manufactured by Osaka Soda Co., Ltd.), L-column ODS (manufactured by Chemicals Evaluation and Research Institute, Japan), Shodex Asahipak (manufactured by Showa Denko K.K.), CORTECS (manufactured by Waters Corporation), and Poroshell (manufactured by Agilent Technologies, Inc.); and TLC plates such as silica gel 60F254 (manufactured by Merck) and Silica Gel Plate (manufactured by Nacalai Tesque, Inc.).

In one example of the present invention, the depletion of the nucleophilic reagent after the reaction of the respiratory sensitization measuring reagent with the test substance may be detected by optical measurement using an ultraviolet detector.

As the ultraviolet detector, a commercially available detector can be used, and examples thereof include those manufactured by Shimadzu Corporation, Waters Corporation, Hitachi, Ltd., and Agilent Technologies, Inc.

In the optical measurement using an ultraviolet detector, the detection wavelength is preferably 200 to 700 nm, more preferably 200 to 400 nm, more preferably 220 to 350 nm, and even more preferably 250 to 300 nm.

In another example of the present invention, the depletion of the nucleophilic reagent after the reaction of the respiratory sensitization measuring reagent with the test substance may be detected by optical measurement using a fluorescence detector.

A molecule in the ground state absorbs excitation light to transition to an excited state. A portion of the absorbed excitation energy is lost in the form of vibrational energy or the like. After nonradiative transition to a lower vibrational level, the molecule returns to the ground state while emitting light, i.e., fluorescence. Optical measurement using a fluorescence detector is thought to be an analytical technique whose sensitivity is generally $10^3$ times or more higher than that of absorptiometry. In addition, since the measurement is made on fluorescent substances, this technique provides excellent selectivity and is used as analytical means for trace amounts of substances. Since fluorescence intensity is proportional to the concentration of the fluorescent substance, quantitative analysis can be carried out by creating a calibration curve. As the fluorescence detector, a commercially available detector can be used, and examples thereof include those manufactured by Shimadzu Corporation, Waters Corporation, Hitachi, Ltd., Agilent Technologies, Inc., and Osaka Soda Co., Ltd.

In the optical measurement using a fluorescence detector, the excitation wavelength is preferably 200 to 350 nm, more preferably 230 to 320 nm, even more preferably 250 to 300 nm, yet more preferably 270 to 300 nm, and particularly preferably 280 to 290 nm. The fluorescence wavelength is preferably 200 to 400 nm, more preferably 300 to 370 nm, even more preferably 300 to 360 nm, and particularly preferably 320 to 350 nm.

The depletion of the N-(arylalkylcarbonyl)cysteine and the depletion of the α-N-(arylalkylcarbonyl)lysine can be calculated from the average peak area of the N-(arylalkylcarbonyl)cysteine and the average peak area of the α-N-(arylalkylcarbonyl)lysine in the optical measurement using an ultraviolet detector or a fluorescence detector by the

following equations.

$$\text{depletion (\% depletion) of N-(arylalkylcarbonyl)cysteine} = [1 - (\text{average peak area of unreacted N-(arylalkylcarbonyl)cysteine after reaction/average peak area of standard N-(arylalkylcarbonyl)cysteine)}] \times 100$$

$$\text{depletion (\% depletion) of } \alpha\text{-N-(arylalkylcarbonyl)lysine} = [1 - (\text{average peak area of unreacted } \alpha\text{-N-(arylalkylcarbonyl)lysine after reaction/average peak area of standard } \alpha\text{-N-(arylalkylcarbonyl)lysine)}] \times 100$$

In the present invention, it is preferably determined whether the test substance is a respiratory sensitizer based on the following criteria.

In the following, [A] represents the depletion of the N-(arylalkylcarbonyl)cysteine, [B] represents the depletion of the $\alpha$-N-(arylalkylcarbonyl)lysine, and [C] represents the average of [A] and [B].

Determination Criterion 1

When the reaction of the N-(arylalkylcarbonyl)cysteine with at least one test substance and the reaction of the $\alpha$-N-(arylalkylcarbonyl)lysine with the at least one test substance are separately carried out, the test substance is determined to be a respiratory sensitizer if

[C] ≥ 4.9% and [B]/[A] ≥ 0.9 are satisfied, or
[B] ≥ 20% is satisfied.

Determination Criterion 2

When the reaction of the N-(arylalkylcarbonyl)cysteine with at least one test substance and the reaction of the $\alpha$-N-(arylalkylcarbonyl)lysine with the at least one test substance are carried out using a mixed solution with a pH of 8.0 including the N-(arylalkylcarbonyl)cysteine and the $\alpha$-N-(arylalkylcarbonyl)lysine, the test substance is determined to be a respiratory sensitizer if

[C] ≥ 4.9% and [B]/[A] ≥ 0.9 are satisfied, or
[B] ≥ 10% is satisfied.

Determination Criterion 3

After the test substance is determined not to be a respiratory sensitizer by the determination based on Determination Criterion 2 described above,

the reaction of the N-(arylalkylcarbonyl)cysteine with the at least one test substance and the reaction of the $\alpha$-N-(arylalkylcarbonyl)lysine with the at least one test substance are carried out using a mixed solution with a pH of 10.2 including the N-(arylalkylcarbonyl)cysteine and the $\alpha$-N-(arylalkylcarbonyl)lysine, and
the test substance is determined to be a respiratory sensitizer if
[B] ≥ 20% is satisfied.

In one example of the present invention, a diisocyanate can be determined to be a respiratory sensitizer by the determination described above. Examples of diisocyanates include, but are not particularly limited to, 1,5-naphthalene diisocyanate, isophorone diisocyanate, methylene bisphenyl diisocyanate, hexamethylene diisocyanate, 2,4-toluene diisocyanate, and 2,6-toluene diisocyanate.

The present invention will be more specifically described with reference to the following examples, although the present invention is not limited to these examples. Examples

Abbreviations in the examples have the following meanings:

EDTA: ethylenediaminetetraacetic acid

NAC: N-[2-(naphthalen-1-yl)acetyl]cysteine
NAL: $\alpha$-N-[2-(naphthalen-1-yl)acetyl]lysine
TFA: trifluoroacetic acid
SD: standard deviation

Test Method

(1) Preparation of Various Solutions

(1-1) 0.1 mmol/LEDTA Aqueous Solution

1) Into a 15 mL conical tube is weighed 37.2 mg of EDTA·2Na·2H$_2$O (manufactured by Dojindo Laboratories), and it is dissolved by adding 10 mL of distilled water (manufactured by Hikari Pharmaceutical Co., Ltd., water for injection in Japanese Pharmacopoeia) using a 25 mL measuring pipette (10 mmol/L EDTA aqueous solution).
2) To a 100 mL container, 49.5 mL of distilled water (manufactured by Hikari Pharmaceutical Co., Ltd., water for injection in Japanese Pharmacopoeia) is added using a 50 mL measuring pipette, and 0.5 mL of the 10 mmol/L EDTA aqueous solution in 1) above is added and mixed so that the solution is diluted 100-fold (0.1 mmol/L EDTA aqueous solution).

(1-2) 100 mmol/L Phosphate Buffer Solution (pH 8.0)

1) Into a 100 mL container is weighed 0.6 g of anhydrous sodium dihydrogen phosphate (manufactured by FUJIFILM Wako Pure Chemical Corporation, Special Grade)), and it is dissolved by adding 50 mL of distilled water (manufactured by Hikari Pharmaceutical Co., Ltd., water for injection in Japanese Pharmacopoeia) using a 50 mL measuring pipette.
2) To a 500 mL container, 300 mL of distilled water (manufactured by Hikari Pharmaceutical Co., Ltd., water for injection in Japanese Pharmacopoeia) is added using a 50 mL (or 100 mL) measuring pipette.
3) After 4.26 g of anhydrous disodium hydrogen phosphate (manufactured by FUJIFILM Wako Pure Chemical Corporation, Special Grade)) is weighed, it is added and dissolved in the distilled water in 2) (manufactured by Hikari Pharmaceutical Co., Ltd., water for injection in Japanese Pharmacopoeia).
4) To the anhydrous disodium hydrogen phosphate solution in 3), 16 mL of the anhydrous sodium dihydrogen phosphate solution in 1) is added using a 25 mL measuring pipette.
5) After 17 mL of the solution in 4) is removed using a 25 mL measuring pipette, 1 mL of the 0.1 mmol/L EDTA aqueous solution is added to the remainder in 4) so that the total volume is 300 mL. The concentration of EDTA in this solution is 0.33 $\mu$mol/L, and the concentration of EDTA in a reaction solution is 0.25 $\mu$mol/L.
6) A portion of the above solution is collected into another container, and the pH is measured with a pH meter to confirm that the pH is within the range of 7.9 to 8.1.

(1-3) 100 mmol/L Phosphate Buffer Solution (pH 10.2)

1) To a 500 mL container, 286 mL of distilled water (manufactured by Hikari Pharmaceutical Co., Ltd., water for injection in Japanese Pharmacopoeia) is added using a 50 mL measuring pipette.
2) After 4.26 g of anhydrous disodium hydrogen phosphate is weighed, it is added and dissolved in the distilled water in 1) (manufactured by Hikari Pharmaceutical Co., Ltd., water for injection in Japanese Pharmacopoeia).
3) Using a 25 mL measuring pipette, 14 mL of a 0.1 mol/L NaOH aqueous solution (manufactured by FUJIFILM Wako Pure Chemical Corporation, Special Grade) is added.
4) A portion of the above solution is collected into another container, and the pH is measured with a pH meter (portable pH meter (HM-20P), Toa Electronics Ltd.) to confirm that the pH is within the range of 10.1 to 10.3.

(1-4) Reaction Stop Solution (2.5% (v/v) TFA Aqueous Solution or 0.5% (v/v) TFA Aqueous Solution)

To 100 mL of distilled water (manufactured by FUJIFILM Wako Pure Chemical Corporation) is added 2.5 mL of TFA (manufactured by FUJIFILM Wako Pure Chemical Corporation, Special Grade) (2.5% (v/v)).

To 100 mL of distilled water (manufactured by FUJIFILM Wako Pure Chemical Corporation) is added 0.5 mL of TFA (manufactured by FUJIFILM Wako Pure Chemical Corporation, Special Grade) (0.5% (v/v)).

(1-5) HPLC Mobile Phase A: 0.1% (v/v) TFAAqueous Solution

To 1 L of distilled water (manufactured by FUJIFILM Wako Pure Chemical Corporation) is added 1.0 mL of TFA.

(1-6) HPLC Mobile Phase B: 0.1% (v/v) TFA Solution in Acetonitrile

To 1 L of HPLC-grade acetonitrile (manufactured by FUJIFILM Wako Pure Chemical Corporation, for HPLC) is added 1.0 mL of TFA.

(2) Preparation of Nucleophilic Reagent Stock Solutions

(2-1) Preparation of NAC Stock Solution

The same stock solution is used in one test. The stock solution is stored in portions that can be used up for each test. A specific example of preparation is shown below.

1) Into a 50 mL container is weighed 11.6 mg ($\pm$0.1 mg) of NAC, and it is dissolved by adding 20 mL of the 100 mmol/L phosphate buffer solution (pH 8.0) using a 25 mL measuring pipette and gently stirring the mixture with a test tube mixer (2 mmol/L).
2) To a 500 mL container, 149.5 mL of the same buffer solution is added using a 50 mL measuring pipette, and 0.5 mL of the above 2 mmol/L NAC solution is added and mixed by inversion so that the solution is diluted 300-fold (6.667 $\mu$mol/L).

(2-2) Preparation of NAL Stock Solution (molecular weight = 314.3 8)

The same stock solution is used in one test. The stock solution is stored in portions that can be used up for each test. A specific example of preparation is shown below.

1) Into a 50 mL container is weighed 12.6 mg ($\pm$0.1 mg) of NAL, and it is dissolved by adding 20 mL of the 100 mmol/L phosphate buffer solution (pH 10.2) using a 25 mL measuring pipette and gently stirring the mixture with a test tube mixer (2 mmol/L NAL solution).
2) To a 500 mL container, 149.5 mL of the same buffer solution is added using a 50 mL measuring pipette, and 0.5 mL of the above 2 mmol/L NAL solution is added and mixed by inversion so that the solution is diluted 300-fold (6.667 $\mu$mol/L).

(2-3) Preparation of NAC/NAL Mixed Stock Solution (pH 8.0)

1) Into a 50 mL container are weighed 11.6 mg ($\pm$0.1 mg) of a NAC preparation and 12.6 mg ($\pm$0.1 mg) of a NAL preparation, and they are dissolved by adding 20 mL of the 100 mmol/L phosphate buffer solution (pH 8.0) using a 25 mL measuring pipette and gently stirring the mixture with a test tube mixer (2 mmol/L).
2) To a 500 mL container, 149.5 mL of the same buffer solution is added using a 50 mL measuring pipette, and 0.5 mL of the above 2 mmol/L NAC solution and 0.5 mL of the above 2 mmol/L NAL solution are added and mixed by inversion so that the solution is diluted 300-fold (6.667 $\mu$mol/L).

(2-4) Preparation of NAC/NAL Mixed Stock Solution (pH 10.2)

1) Into a 50 mL container are weighed 11.6 mg ($\pm$0.1 mg) of a NAC preparation and 12.6 mg ($\pm$0.1 mg) of a NAL preparation, and they are dissolved by adding 20 mL of the 100 mmol/L phosphate buffer solution (pH 10.2) using a 25 mL measuring pipette and gently stirring the mixture with a test tube mixer (2 mmol/L).
2) To a 500 mL container, 149.5 mL of the same buffer solution is added using a 50 mL measuring pipette, and 0.5 mL of the above 2 mmol/L NAC solution and 0.5 mL of the above 2 mmol/L NAL solution are added and mixed by inversion so that the solution is diluted 300-fold (6.667 μmol/L).

(3) Preparation of Test Substance Solutions

One solvent from which a 1 mmol/L test substance solution can be prepared is selected according to the following order of priority: water, acetonitrile, acetone, and 5% DMSO solution in acetonitrile. When water, acetonitrile, or acetone is selected, a 20 mmol/L test substance solution is first prepared. A suitable amount of the test substance is weighed and completely dissolved by adding the solvent to prepare a 20 mmol/L solution. A portion of the 20 mmol/L solution is then collected and diluted 20-fold with the same solvent to prepare a 1 mmol/L test substance solution. When a 5% DMSO solution in acetonitrile is selected, a 20 mmol/L DMSO solution is prepared in the same manner as above. A portion of the 20 mmol/L solution is then collected and diluted 20-fold with acetonitrile to prepare a 1 mmol/L test substance solution.

(4) Reaction

(4-1) Addition

Test substance solutions are prepared on a 96-well plate (U96 PP-0.5 ML NATURAL, Thermo (NUNC)), mainly using a 12-channel pipette, and the reagents are added in the following amounts:

Nucleophilic reagents (NAC and NAL): 150 μL
For the NAC/NAL mixed stock solution (pH 8.0), 150 μL of the solution in (2-3) above is added. For the NAC/NAL mixed stock solution (pH 10.2), 150 μL of the solution in (2-4) above is added.
Test substance solution: 50 μL

(4-2) Reaction

The plate is firmly sealed with a plate seal (resistant embossed seal, Shimadzu GLC Ltd.) and is shaken on a plate shaker (Titramax 100, Heidolph Instruments). After spinning down in a centrifuge, the solutions are incubated at 25°C in a light-shielded state for 24 hours.

(4-3) Reaction Stop

After incubation for 24 hours, the plate seal is removed, and 50 μL of the reaction stop solution (2.5% (v/v) TFA) is added to each sample to stop the reaction.

(5) HPLC Measurement

The HPLC measurement conditions for the nucleophilic reagents are shown below.

Table 1

Table 1: HPLC measurement conditions

| HPLC instrument | LC-20A (Prominence) series (Shimadzu Corporation) |
|---|---|
| Column | CAPCELL CORE C18 column (3.0 × 150 mm, 2.7 μm) (Osaka Soda Co., Ltd.) |
| Detector | UV detection: SPD-M20A (Shimadzu Corporation) |
| Detection wavelength | UV detection: 281 nm |
| Column temperature | 40°C |
| Sample temperature | 25°C |
| Injection volume | 10-20 μl |

(continued)

| Eluent | A: water (0.1% trifluoroacetic acid) | | | | |
| | B: acetonitrile (0.1% trifluoroacetic acid) | | | | |
| Measurement time | 20 minutes | | | | |
| Elution conditions | [NAC] | Time (min.) | Flow rate (ml/min.) | %A | %B |
| | | 0.0 | 0.3 | 70 | 30 |
| | | 9.5 | 0.3 | 45 | 55 |
| | | 10.0 | 0.3 | 0 | 100 |
| | | 13.0 | 0.3 | 0 | 100 |
| | | 13.5 | 0.3 | 70 | 30 |
| | | 20.0 | End | | |
| | [NAL] | Time (mm.) | Flow rate (ml/min.) | %A | %B |
| | | 0.0 | 0.3 | 80 | 20 |
| | | 9.5 | 0.3 | 55 | 45 |
| | | 10.0 | 0.3 | 0 | 100 |
| | | 13.0 | 0.3 | 0 | 100 |
| | | 13.5 | 0.3 | 80 | 20 |
| | | 20.0 | End | | |

UV Detection

In UV detection, the area of a peak detected at 281 nm is determined.

(6) Data Analysis

(6-1) Calculation of Depletion

The depletion of NAC or NAL is calculated from the average peak area of unreacted NAC or NAL after the reaction and the average peak area of standard (before the reaction) NAC or NAL (n = 3 for each) by the following equations:

$$\text{NAC depletion (\% depletion)} = [1 - (\text{average peak area of unreacted NAC after reaction/average peak area of standard (before reaction) NAC})] \times 100$$

$$\text{NAL depletion (\% depletion)} = [1 - (\text{average peak area of unreacted NAL after reaction/average peak area of standard (before reaction) NAL})] \times 100$$

The average peak area of unreacted NAC after the reaction is the average area of unreacted NAC after the reaction of the test substance with the nucleophilic reagent (NAC) measured three times, and the average peak area of standard (before the reaction) NAC is the average area of standard (before the reaction) NAC measured three times.
The average peak area of unreacted NAL after the reaction is the average area of unreacted NAL after the reaction of the test substance with the nucleophilic reagent (NAL) measured three times, and the average peak area of standard (before the reaction) NAL is the average area of standard (before the reaction) NAL measured three times.

(6-2) Average Score

The average of the NAC depletion and the NAL depletion (average score) is calculated. The score is rounded to one decimal place.

$$\text{Average of NAC and NAL depletions (average score)} = (\text{NAC depletion} + \text{NAL depletion})/2$$

Using the NAC depletion [A], the NAL depletion [B], and the average [C] of [A] and [B], evaluation is carried out by any of the following methods (1) and (2).

(1) When NAC and NAL are separately prepared and used to carry out evaluation, the test substance is determined to be a respiratory sensitizer if the following conditions are satisfied:

average score $[C] \geq 4.9\%$ and $[B]/[A] \geq 0.9$, or
$[B] \geq 20\%$

(2) When a mixed solution of NAC and NAL (pH 8.0) is used to carry out evaluation, the test substance is determined to be a respiratory sensitizer if the following conditions are satisfied:

average score $[C] \geq 4.9\%$ and $[B]/[A] \geq 0.9$, or
$[B] \geq 10\%$

After the test substance is determined not to be a respiratory sensitizer by the determination in (2) above,
(3) a mixed solution of NAC and NAL (pH 10.2) is used to carry out evaluation, and the test substance is determined to be a respiratory sensitizer if the following conditions are satisfied:
$[B] \geq 20\%$

Example 1

The 13 respiratory sensitizers listed in the table below were evaluated under ADRA and DPRA test conditions. As the results of DPRA, the values described in Lalko JF, Kimber I, Gerberick GF, Foertsch LM, Api AM, Dearman RJ. (2012). The direct peptide reactivity assay: selectivity of chemical respiratory allergens. Toxicological Sciences, 129(2), 421-431. were used.

Test Substances and Preparation of Solutions:

For ADRA, 1 mmol/L solutions of the following 13 sensitizers were prepared and used in the test.

Reaction Conditions:

For ADRA, the respiratory sensitizers were reacted with NAC and NAL under the conditions described in "(4) Reaction" above (the conditions where NAC and NAL are separately reacted).

Measurement Conditions:

For ADRA, the depletions (%) of the nucleophilic reagents were determined under the HPLC measurement conditions described in "(5) HPLC Measurement" above.

Results:

The results of the tests carried out for the 13 test substances in accordance with the above test methods are summarized below.
In ADRA, the 13 respiratory sensitizers were correctly determined to be respiratory sensitizers. In DPRA, on the other hand, the four diisocyanates were not correctly evaluated. It was found that diisocyanates, which cannot be evaluated by DPRA, can also be correctly evaluated by ADRA.

Table 2

| Respiratory sensitizer | ADRA | | | | | | DPRA | | | | |
|---|---|---|---|---|---|---|---|---|---|---|---|
| | NAC depletion (%) [A] | NAL depletion (%) [B] | Determination Criterion 1 Average % depletion | Determination Criterion 2 NAL/NAC [B]/[A] | Determination Criterion 3 NAL depletion (%) | Prediction | Cysteine peptide depletion (%) [A] | Lysine peptide depletion (%) [B] | Determination Criterion 1 Average % depletion | Determination Criterion 2 [B]/[A] | Prediction |
| 1 | 1.3 | 57.0 | 29.2 | 42.8 | 57.0 | Pos. | 8.1 | 86.3 | 47.2 | 10.7 | Pos. |
| 2 | 100.0 | 89.6 | 94.8 | 0.9 | 89.6 | Pos. | 54.0 | 93.0 | 73.5 | 1.7 | Pos. |
| 3 | 0.3 | 96.9 | 48.6 | 323.0 | 96.9 | Pos. | 10.9 | 94.0 | 52.5 | 8.6 | Pos. |
| 4 | 1.9 | 97.0 | 49.4 | 52.3 | 97.0 | Pos. | 8.9 | 95.3 | 52.1 | 10.7 | Pos. |
| 5 | 37.3 | 98.1 | 67.7 | 2.6 | 98.1 | Pos. | 83.5 | 25.1 | 54.3 | 0.3 | Neg |
| 6 | 67.5 | 69.8 | 68.6 | 1.0 | 69.8 | Pos. | 100.0 | 35.0 | 67.5 | 0.4 | Neg |
| 7 | 25.0 | 98.3 | 61.7 | 3.9 | 98.3 | Pos. | 54.5 | 23.0 | 38.8 | 0.4 | Neg |
| 8 | 26.0 | 98.5 | 62.3 | 3.8 | 98.5 | Pos. | 87.4 | 26.3 | 56.9 | 0.3 | Neg |
| 9 | 0.8 | 53.1 | 26.9 | 66.4 | 53.1 | Pos. | 35.5 | 79.6 | 57.6 | 2.2 | Pos. |
| 10 | 98.4 | 100.0 | 99.2 | 1.0 | 100.0 | Pos. | 77.3 | 99.5 | 88.4 | 1.3 | Pos. |
| 11 | 100.0 | 100.0 | 100.0 | 1.0 | 100.0 | Pos. | 93.5 | 97.3 | 95.4 | 1.0 | Pos. |
| 12 | 100.0 | 97.8 | 98.9 | 1.0 | 97.8 | Pos. | 99.5 | 98.9 | 99.2 | 1.0 | Pos. |
| 13 | 100.0 | 88.1 | 94.0 | 0.9 | 88.1 | Pos. | 74.1 | 99.3 | 86.7 | 1.3 | Pos. |

Respiratory sensitizers in Table 2

1: hexahydrophthalic anhydride
2: maleic anhydride
3: phthalic anhydride
4: trimellitic anhydride
5: methylene bisphenyl diisocyanate
6: hexamethylene diisocyanate
7: 2,4-toluene diisocyanate
8: 2,6-toluene diisocyanate
9: glutaraldehyde
10: Black GR Reactive
11: Orange-3R Reactive
12: chloramine-T
13: cyanuric chloride

Note: In the "prediction of respiratory sensitizer" column, Pos indicates a substance predicted as a respiratory sensitizer, and Neg indicates a substance predicted as a non-sensitizer.

Example 2

The 5 respiratory sensitizers and 27 non-respiratory sensitizers listed in the table below were evaluated under normal ADRA test conditions (the conditions where NAC and NAL are separately reacted).

Test Substances and Preparation of Solutions:

In the test, 1 mmol/L solutions of the following 32 test substances were prepared and used.

Measurement Conditions:

The depletions (%) of the nucleophilic reagents were determined under the HPLC measurement conditions described in "(5) HPLC Measurement" above.

Results:

The results of the test carried out for the 32 test substances (5 respiratory sensitizers and 27 non-sensitizers) in accordance with the above test method are summarized below.

All 5 respiratory sensitizers were correctly determined to be respiratory sensitizers, and all 27 non-sensitizers were correctly determined to be non-sensitizers.

Table 3

| Test substance | Respiratory sensitizer | ADRA | | | | | | | |
| | | NAC | | NAL | | Average depletion (%) | NAL/NAC | NAL depletion (%) | Prediction of respiratory sensitization |
| | | Depletion (%) | SD | Depletion (%) | SD | | | | |
| 1 | Yes | 0.8 | 0.8 | 43.4 | 0.7 | 22.1 | 51.9 | 43.4 | Pos |
| 2 | Yes | 21.7 | 0.3 | 100.0 | 0.0 | 60.8 | 4.6 | 100.0 | Pos |
| 3 | Yes | 70.0 | 0.1 | 62.3 | 0.1 | 66.2 | 0.9 | 62.3 | Pos |
| 4 | Yes | 100.0 | 0.0 | 21.5 | 0.4 | 60.8 | 0.2 | 21.5 | Pos |
| 5 | Yes | 17.5 | 0.7 | 57.7 | 3.0 | 37.6 | 3.3 | 57.7 | Pos |
| 6 | No | 0.3 | 0.3 | 0.7 | 1.2 | 0.5 | 2.1 | 0.7 | Neg |
| 7 | No | 0.2 | 0.3 | 0.9 | 0.9 | 0.5 | 6.0 | 0.9 | Neg |
| 8 | No | 0.0 | 0.0 | 1.7 | 1.4 | 0.9 | - | 1.7 | Neg |
| 9 | No | 0.0 | 0.0 | 1.2 | 0.5 | 0.6 | - | 1.2 | Neg |
| 10 | No | 2.4 | 0.5 | 2.2 | 0.6 | 2.3 | 0.9 | 2.2 | Neg |
| 11 | No | 0.1 | 0.2 | 0.8 | 0.5 | 0.5 | 6.0 | 0.8 | Neg |
| 12 | No | 0.0 | 0.0 | 0.0 | 0.0 | 0.0 | 0.0 | 0.0 | Neg |
| 13 | No | 0.0 | 0.0 | 1.0 | 0.7 | 0.5 | - | 1.0 | Neg |
| 14 | No | 0.0 | 0.0 | 0.4 | 0.6 | 0.2 | - | 0.4 | Neg |
| 15 | No | 0.0 | 0.0 | 0.7 | 0.7 | 0.4 | - | 0.7 | Neg |
| 16 | No | 0.0 | 0.0 | 2.8 | 0.6 | 1.4 | - | 2.8 | Neg |
| 17 | No | 0.1 | 0.1 | 2.4 | 1.6 | 1.2 | 39.0 | 2.4 | Neg |
| 18 | No | 1.3 | 0.6 | 0.3 | 0.6 | 0.8 | 0.2 | 0.3 | Neg |
| 19 | No | 3.3 | 0.4 | 3.3 | 0.6 | 3.3 | 1.0 | 3.3 | Neg |
| 20 | No | 1.4 | 0.3 | 2.1 | 0.8 | 1.7 | 1.6 | 2.1 | Neg |
| 21 | No | 0.1 | 0.1 | 2.8 | 0.6 | 1.5 | 26.0 | 2.8 | Neg |
| 22 | No | 0.1 | 0.2 | 2.2 | 0.7 | 1.1 | 19.8 | 2.2 | Neg |
| 23 | No | 0.0 | 0.0 | 1.4 | 0.7 | 0.7 | 65.8 | 1.4 | Neg |

(continued)

| Test substance | Respiratory sensitizer | ADRA | | | | | | | |
|---|---|---|---|---|---|---|---|---|---|
| | | NAC | | NAL | | Average depletion (%) | NAL/NAC | NAL depletion (%) | Prediction of respiratory sensitization |
| | | Depletion (%) | SD | Depletion (%) | SD | | | | |
| 24 | No | 0.0 | 0.0 | 1.8 | 1.4 | 0.9 | 355.3 | 1.8 | Neg |
| 25 | No | 0.1 | 0.1 | 1.3 | 0.5 | 0.7 | 15.5 | 1.3 | Neg |
| 26 | No | 0.0 | 0.0 | 1.3 | 0.7 | 0.6 | - | 1.3 | Neg |
| 27 | No | 0.1 | 0.2 | 1.9 | 3.1 | 1.0 | 20.0 | 1.9 | Neg |
| 28 | No | 0.0 | 0.0 | 0.4 | 0.6 | 0.2 | - | 0.4 | Neg |
| 29 | No | 0.5 | 0.4 | 0.4 | 0.2 | 0.5 | 0.9 | 0.4 | Neg |
| 30 | No | 1.5 | 0.3 | 1.9 | 1.5 | 1.7 | 1.2 | 1.9 | Neg |
| 31 | No | 0.5 | 0.5 | 3.9 | 2.4 | 2.2 | 7.2 | 3.9 | Neg |
| 32 | No | 0.9 | 0.4 | 0.0 | 0.0 | 0.4 | 0.0 | 0.0 | Neg |

Names of test substances in Table 3

1: chlorendic anhydride
2: 1,5-naphthalene diisocyanate
3: isophorone diisocyanate
4: ninhydrin
5: phenyl isothiocyanate
6: glycerol
7: hexane
8: diethyl phthalate
9: octanoic acid
10: 2-hydroxypropyl methacrylate
11: 1-butanol
12: 4-hydroxybenzoic acid
13: 6-methylcoumarin
14: methyl salicylate
15: chlorobenzene
16: lactic acid
17: 1-bromobutane
18: 2-acetylcyclohexanone
19: 4'-methoxyacetophenone
20: ethyl vanillin
21: isopropanol
22: propylene glycol
23: sulfanilamide
24: isopropyl myristate
25: methylparaben
26: nonanoic acid
27: propylparaben
28: salicylic acid
29: sulfanilic acid
30: vanillin
31: coumarin
32: vinylidene dichloride

Note:

In the "respiratory sensitizer" column in the table, "Yes" indicates a respiratory sensitizer, and "No" indicates a non-sensitizer.
In the "prediction of respiratory sensitizer" column, "Pos" indicates a substance predicted as a respiratory sensitizer, and "Neg" indicates a substance predicted as a non-sensitizer.

Example 3

In Examples 1 and 2, as in conventional ADRA test conditions, reaction solutions of NAC and NAL were separately prepared and each subjected to HPLC measurement for evaluation. However, if NAC and NAL are mixed and reacted in one reaction solution, evaluation can be carried out by one HPLC measurement, which leads to a considerable improvement in efficiency. Accordingly, the 18 respiratory sensitizers used in Examples 1 and 2 were evaluated under test conditions where the NAC/NAL mixed stock solution (pH 8.0) in "(2-3) Preparation of NAC/NAL Mixed Stock Solution (pH 8.0)" and "(4-1)" of "Test Method" above was used.
The above results show that the 27 non-respiratory sensitizers almost did not react with NAC or NAL; therefore, they were not evaluated since the above results for NAC or NAL will probably be reproduced when evaluation is carried out in a mixed solution of NAC and NAL.

Test Substances and Preparation of Solutions:

In the test, 1 mmol/L solutions of the following 18 test substances were prepared and used.

Measurement Conditions:

The depletions (%) of the nucleophilic reagents were determined under the HPLC measurement conditions described in "(5) HPLC Measurement" above.

Results:

As above, the results of the test carried out for the 18 test substances in accordance with the above test method are summarized below.

Table 4

ADRA (NAC/NAL mixture: pH = 8.0)

| Test substance | Respiratory sensitizer | NAC Depletion (%) | NAC SD | NAL Depletion (%) | NAL SD | Average depletion (%) | NAL/NAC | NAL depletion (%) | Prediction of respiratory sensitization |
|---|---|---|---|---|---|---|---|---|---|
| 1 | Yes | 1.1 | 0.4 | 96.6 | 0.6 | 48.8 | 88.5 | 96.6 | Pos |
| 2 | Yes | 1.3 | 1.1 | 100.0 | 0.0 | 50.6 | 79.2 | 100.0 | Pos |
| 3 | Yes | 100.0 | 0.0 | 96.9 | 0.5 | 98.5 | 1.0 | 96.9 | Pos |
| 4 | Yes | 8.6 | 0.2 | 70.0 | 1.0 | 39.3 | 8.1 | 70.0 | Pos |
| 5 | Yes | 0.0 | 0.0 | 50.1 | 0.8 | 25.1 | - | 50.1 | Pos |
| 6 | Yes | 24.1 | 1.3 | 92.0 | 0.7 | 58.1 | 3.8 | 92.0 | Pos |
| 7 | Yes | 27.9 | 1.0 | 81.1 | 1.4 | 54.5 | 2.9 | 81.1 | Pos |
| 8 | Yes | 23.9 | 0.8 | 61.8 | 4.7 | 42.8 | 2.6 | 61.8 | Pos |
| 9 | Yes | 40.0 | 1.5 | 70.4 | 0.5 | 55.2 | 1.8 | 70.4 | Pos |
| 10 | Yes | 68.7 | 0.4 | 15.9 | 0.3 | 42.3 | 0.2 | 15.9 | Pos |
| 11 | Yes | 67.2 | 0.7 | 24.0 | 1.4 | 45.6 | 0.4 | 24.0 | Pos |
| 12 | Yes | 6.2 | 0.5 | 0.0 | 0.0 | 3.1 | 0.0 | 0.0 | Neg |
| 13 | Yes | 100.0 | 0.0 | 96.2 | 0.1 | 98.1 | 1.0 | 96.2 | Pos |
| 14 | Yes | 100.0 | 0.0 | 43.6 | 1.8 | 71.8 | 0.4 | 43.6 | Pos |
| 15 | Yes | 98.3 | 0.3 | 2.0 | 0.6 | 50.1 | 0.0 | 2.0 | Neg |
| 16 | Yes | 100.0 | 0.0 | 47.0 | 0.3 | 73.5 | 0.5 | 47.0 | Pos |
| 17 | Yes | 96.3 | 0.4 | 9.6 | 0.4 | 53.0 | 0.1 | 9.6 | Neg |
| 18 | Yes | 16.8 | 1.4 | 0.2 | 0.4 | 8.5 | 0.0 | 0.2 | Neg |

Names of test substances in Table 4

1: phthalic anhydride

2: trimellitic anhydride

3: maleic anhydride

4: hexahydrophthalic anhydride

5: chlorendic anhydride

6: 1,5-naphthalene diisocyanate

7: 2,6-toluene diisocyanate

8: 2,4-toluene diisocyanate

9: methylene bisphenyl diisocyanate

10: isophorone diisocyanate

11: hexamethylene diisocyanate

12: glutaraldehyde

13: chloramine-T

14: cyanuric chloride

15: ninhydrin

16: Black GR Reactive

17: Orange-3R Reactive

18: phenyl isothiocyanate

Note:

In the "respiratory sensitizer" column in the table, "Yes" indicates a respiratory sensitizer. In the "prediction of respiratory sensitizer" column, "Pos" indicates a substance predicted as a respiratory sensitizer, and "Neg" indicates a substance predicted as a non-sensitizer.

It was found that, of the 18 respiratory sensitizers, four respiratory sensitizers (Nos. 12, 15, 17, and 18) were not correctly determined, but 14 respiratory sensitizers were correctly determined to be respiratory sensitizers. It was found that the prediction accuracy for the test substances including the 27 non-sensitizers was 91.1%. The above results show that the test method in which NAC and NAL are mixed and reacted in one reaction solution is a test method capable of satisfactorily determining a respiratory sensitizer.

Example 4

Of the 18 respiratory sensitizers used in Example 3, five substances including the four substances that were not correctly predicted as respiratory sensitizers and isophorone diisocyanate, for which the NAL depletion was relatively low, i.e., 15.9%, were evaluated in a buffer solution with a pH of 10.2 as normal NAL reaction conditions.

Test Substances and Preparation of Solutions:

In the test, 1 mmol/L solutions of the following five test substances were prepared and used.

Measurement Conditions:

The depletions (%) of the nucleophilic reagents were determined under the HPLC measurement conditions described in "(5) HPLC Measurement" above.

Results:

The results of the test carried out for the five test substances in accordance with the above test method are summarized below.

Table 5

| Test substance | Respiratory sensitizer | ADRA (NAC/NAL mixture: pH = 10.2) | | | | | |
| | | NAC | | NAL | | Average depletion (%) | Prediction of respiratory sensitization |
| | | Depletion (%) | SD | Depletion (%) | SD | | |
| 1 | Yes | - | - | 63.2 | 1.7 | >31.6 | Pos |
| 2 | Yes | - | - | 21.9 | 0.4 | >10.9 | Pos |
| 3 | Yes | - | - | 25.2 | 0.4 | >12.6 | Pos |
| 4 | Yes | - | - | 100.0 | 0.0 | >50.0 | Pos |
| 5 | Yes | - | - | 58.6 | 0.5 | >29.3 | Pos |

Names of test substances in Table 5

1: isophorone diisocyanate
2: glutaraldehyde
3: ninhydrin
4: Orange-3R Reactive
5: phenyl isothiocyanate

Note:

In the "respiratory sensitizer" column in the table, "Yes" indicates a respiratory sensitizer. In the "prediction of respiratory sensitizer" column in the table, "Pos" indicates a substance predicted as a respiratory sensitizer. In the "NAC depletion" column, "-" indicates that evaluation was impossible since NAC was almost completely oxidized at a pH of 10.2.

In the test system using the buffer solution with a pH of 10.2 in which NAC and NAL were mixed, the five respiratory sensitizers that were erroneously predicted as non-sensitizers in Example 3 were also correctly predicted as respiratory sensitizers. The above results show that the test method in which NAC and NAL are mixed and reacted in one reaction solution is a test method capable of determining a respiratory sensitizer if the buffer solution used is appropriately selected.

Whereas the depletion of NAL for glutaraldehyde was 53.1% in Example 1, it decreased to 21.9% in Example 4. A possible reason for this is that, since Example 4 was evaluated at the same pH as Example 1 but in a mixed solution containing NAC, NAC had a reaction suppressing effect.

**Claims**

**1.** A method for measuring respiratory sensitization, comprising:

reacting a N-(arylalkylcarbonyl)cysteine with at least one test substance;

reacting an $\alpha$-N-(arylalkylcarbonyl)lysine with the at least one test substance;

detecting an amount of the N-(arylalkylcarbonyl)cysteine or a product thereof after the reaction and an amount of the $\alpha$-N-(arylalkylcarbonyl)lysine or a product thereof after the reaction by optical measurement using an ultraviolet detector or a fluorescence detector; and

determining respiratory sensitization from a ratio of a reactivity of the $\alpha$-N-(arylalkylcarbonyl)lysine with the test substance to a reactivity of the N-(arylalkylcarbonyl)cysteine with the test substance or from the reactivity of the $\alpha$-N-(arylalkylcarbonyl)lysine with the test substance.

2. The method for measuring respiratory sensitization according to claim 1, wherein the N-(arylalkylcarbonyl)cysteine and the $\alpha$-N-(arylalkylcarbonyl)lysine are compounds that exhibit absorption in a wavelength range of 200 to 700 nm and that have a molar absorption coefficient of 10 L/mol cm or more and 500,000 L/mol cm or less at a maximal absorption wavelength, or are compounds that emit fluorescence at 200 to 400 nm at an excitation wavelength of 200 to 350 nm.

3. The method for measuring respiratory sensitization according to claim 1 or 2, wherein the N-(arylalkylcarbonyl)cysteine is N-(2-phenylacetyl)cysteine or N-[2-(naphthalen-1-yl)acetyl]cysteine.

4. The method for measuring respiratory sensitization according to any one of claims 1 to 3, wherein the $\alpha$-N-(arylalkylcarbonyl)lysine is $\alpha$-N-(2-phenylacetyl)lysine or $\alpha$-N-[2-(naphthalen-1-yl)acetyl]lysine.

5. The method for measuring respiratory sensitization according to any one of claims 1 to 4, wherein the N-(arylalkylcarbonyl)cysteine and the $\alpha$-N-(arylalkylcarbonyl)lysine are each prepared at a concentration of 0.1 $\mu$mol/L to 100 $\mu$mol/L.

6. The method for measuring respiratory sensitization according to any one of claims 1 to 5, wherein

reacting the N-(arylalkylcarbonyl)cysteine with the at least one test substance comprises reacting a solution of the N-(arylalkylcarbonyl)cysteine with a solution of the test substance in water, an organic solvent, or a mixture thereof at a concentration of 0.1 mmol/L to 10 mmol/L if a molecular weight of the test substance is known or at a concentration of 0.05 mg/mL to 10 mg/mL if the molecular weight of the test substance is unknown, and reacting the $\alpha$-N-(arylalkylcarbonyl)lysine with the at least one test substance comprises reacting a solution of the $\alpha$-N-(arylalkylcarbonyl)lysine with a solution of the test substance in water, an organic solvent, or a mixture thereof at a concentration of 0.1 mmol/L to 10 mmol/L if a molecular weight of the test substance is known or at a concentration of 0.05 mg/mL to 10 mg/mL if the molecular weight of the test substance is unknown.

7. The method for measuring respiratory sensitization according to any one of claims 1 to 6, further comprising chromatographing a product obtained by reacting the N-(arylalkylcarbonyl)cysteine with the at least one test substance and a product obtained by reacting the $\alpha$-N-(arylalkylcarbonyl)lysine with the at least one test substance.

8. The method for measuring respiratory sensitization according to any one of claims 1 to 7, wherein a detection wavelength in the optical measurement using an ultraviolet detector is 200 to 700 nm.

9. The method for measuring respiratory sensitization according to any one of claims 1 to 8, wherein, in the optical measurement using a fluorescence detector, an excitation wavelength is 200 to 350 nm, and a fluorescence wavelength is 200 to 400 nm.

10. The method for measuring respiratory sensitization according to any one of claims 1 to 9, wherein the at least one test substance is at least one of a perfume, an essential oil, a polymer compound, a medicine, an agricultural chemical, a food, a chemical product, or a plant extract made of a naturally derived component.

11. The method for measuring respiratory sensitization according to any one of claims 1 to 10, wherein a depletion of the N-(arylalkylcarbonyl)cysteine and a depletion of the $\alpha$-N-(arylalkylcarbonyl)lysine are calculated from an average peak area of the N-(arylalkylcarbonyl)cysteine and an average peak area of the $\alpha$-N-(arylalkylcarbonyl)lysine in the optical measurement using an ultraviolet detector or a fluorescence detector by the following equations:

depletion (% depletion) of N-(arylalkylcarbonyl)cysteine = [1 - (average peak area of unreacted N-(arylalkylcarbonyl)cysteine after reaction/average peak area of standard N-(arylalkylcarbonyl)cysteine)] × 100

depletion (% depletion) of α-N-(arylalkylcarbonyl)lysine = [1 - (average peak area of unreacted α-N-(arylalkylcarbonyl)lysine after reaction/average peak area of standard α-N-(arylalkylcarbonyl)lysine)] × 100

**12.** The method for measuring respiratory sensitization according to claim 11, wherein

the reaction of the N-(arylalkylcarbonyl)cysteine with the at least one test substance and the reaction of the α-N-(arylalkylcarbonyl)lysine with the at least one test substance are separately carried out, and
the test substance is determined to be a respiratory sensitizer if
[C] ≥ 4.9% and [B]/[A] ≥ 0.9 are satisfied, or
[B] ≥ 20% is satisfied,

where [A] represents the depletion of the N-(arylalkylcarbonyl)cysteine, [B] represents the depletion of the α-N-(arylalkylcarbonyl)lysine, and [C] represents an average of [A] and [B].

**13.** The method for measuring respiratory sensitization according to claim 11, wherein

the reaction of the N-(arylalkylcarbonyl)cysteine with the at least one test substance and the reaction of the α-N-(arylalkylcarbonyl)lysine with the at least one test substance are carried out using a mixed solution with a pH of 8.0 including the N-(arylalkylcarbonyl)cysteine and the α-N-(arylalkylcarbonyl)lysine, and
the test substance is determined to be a respiratory sensitizer if
[C] ≥ 4.9% and [B]/[A] ≥ 0.9 are satisfied, or
[B] ≥ 10% is satisfied,

where [A] represents the depletion of the N-(arylalkylcarbonyl)cysteine, [B] represents the depletion of the α-N-(arylalkylcarbonyl)lysine, and [C] represents an average of [A] and [B].

**14.** The method for measuring respiratory sensitization according to claim 13, wherein

after the test substance is determined not to be a respiratory sensitizer by the determination in claim 13,
the reaction of the N-(arylalkylcarbonyl)cysteine with the at least one test substance and the reaction of the α-N-(arylalkylcarbonyl)lysine with the at least one test substance are carried out using a mixed solution with a pH of 10.2 including the N-(arylalkylcarbonyl)cysteine and the α-N-(arylalkylcarbonyl)lysine, and
the test substance is determined to be a respiratory sensitizer if [B] ≥ 20% is satisfied.

**15.** The method for measuring respiratory sensitization according to any one of claims 1 to 14, wherein a diisocyanate is determined to be a respiratory sensitizer by the determination.

**16.** A respiratory sensitization measuring reagent for measuring respiratory sensitization of at least one test substance by optical measurement using an ultraviolet detector, the respiratory sensitization measuring reagent comprising a N-(arylalkylcarbonyl)cysteine or an α-N-(arylalkylcarbonyl)lysine as a main measuring agent.

**INTERNATIONAL SEARCH REPORT**

| | International application No. |
|---|---|
| | PCT/JP2021/012167 |

**A. CLASSIFICATION OF SUBJECT MATTER**
G01N 33/483(2006.01)i; G01N 33/52(2006.01)i; G01N 21/64(2006.01)i; G01N 21/78(2006.01)i
FI: G01N21/78 C; G01N33/483 C; G01N33/52 C; G01N21/64 Z
According to International Patent Classification (IPC) or to both national classification and IPC

**B. FIELDS SEARCHED**

Minimum documentation searched (classification system followed by classification symbols)
G01N33/483; G01N33/52; G01N21/64; G01N21/78; C12Q1/00-3/00

Documentation searched other than minimum documentation to the extent that such documents are included in the fields searched
| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| A | LALKO, J. F. et al, "The Direct Peptide Reactivity Assay: Selectivity of Chemical Respiratory Allergens", Toxicological Sciences, 19 June 2012, vol. 129, pp. 421-431, doi:10.1093/toxsci/kfs205 whole document | 1-16 |
| A | ARTS, J., "How to assess respiratory sensitization of low molecular weight chemicals?", International Journal of Hygiene and Environmental Health, 12 February 2020, vol. 225, Article No. 113469, pp. 1-8, doi:10.1016/j.ijheh.2020.113469 pp. 5-6, "4.2.2. In chemico/in vitro sensitization testing" | 1-16 |

☒ Further documents are listed in the continuation of Box C.    ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 01 June 2021 (01.06.2021) | 08 June 2021 (08.06.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

| | | **INTERNATIONAL SEARCH REPORT** | International application No. |
|---|---|---|---|
| | | | PCT/JP2021/012167 |

C (Continuation).   DOCUMENTS CONSIDERED TO BE RELEVANT

| Category* | Citation of document, with indication, where appropriate, of the relevant passages | Relevant to claim No. |
|---|---|---|
| A | DIK, S. et al., "Can the Direct Peptide Reactivity Assay Be Used for the Identification of Respiratory Sensitization Potential of Chemicals?", Toxicological Sciences, 29 July 2016, vol. 153, no. 2, pp. 361-371, doi:10.1093/toxsci/kfw130 whole document | 1-16 |
| A | NORTH, C. M. et al., "Developing a framework for assessing chemical respiratory sensitization: A workshop report", Regulatory Toxicology and Pharmacology, 07 July 2016, vol. 80, pp. 295-309, doi:10.1016/j_yrtph.2016.06.006 Whole document | 1-16 |
| A | HEMMING, J. D. C. et al., "Application of the direct peptide reactivity assay (DPRA) to inorganic compounds: a case study of platinum species", Toxicology Research, 20 November 2019, vol. 8, pp. 802-814, doi:10.1039/c9tx00242a whole document | 1-16 |
| A | MIZOGUCHI, I. et al., "Prediction of Chemical Respiratory and Contact Sensitizers by OX40L Expression in Dendritic Cells Using a Novel 3D Coculture System", Frontiers in Immunology, 04 August 2017, vol. 8, Article No. 929, pp. 1-12, doi: 10.3389/fimmu.2017.00929 whole document | 1-16 |
| A | JP 2018-505655 A (SENZAGEN AB) 01 March 2018 (2018-03-01) | 1-16 |
| A | US 2015/0111771 A1 (SENZAGEN AB) 23 April 2015 (2015-04-23) | 1-16 |
| A | JP 2011-059102 A (FUJIFILM CORPORATION) 24 March 2011 (2011-03-24) | 1-16 |
| A | JP 2014-037995 A (FUJIFILM CORPORATION) 27 February 2014 (2014-02-27) | 1-16 |

Form PCT/ISA/210 (continuation of second sheet) (January 2015)

| International application No. |
| --- |
| PCT/JP2021/012167 |

| Patent Documents referred in the Report | Publication Date | Patent Family | Publication Date |
| --- | --- | --- | --- |
| JP 2018-505655 A | 01 Mar. 2018 | US 2017/0285010 A1<br>WO 2016/083604 A1<br>EP 3224618 A1<br>CA 2964294 A1<br>KR 10-2017-0102874 A<br>CN 107250793 A | |
| US 2015/0111771 A1 | 23 Apr. 2015 | US 2017/0283874 A1<br>WO 2013/160882 A1<br>EP 2841599 A1<br>CA 2871350 A1<br>CN 104685067 A | |
| JP 2011-059102 A | 24 Mar. 2011 | (Family: none) | |
| JP 2014-037995 A | 27 Feb. 2014 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001059102 A
- JP 2014037995 A
- JP 2011059102 A

**Non-patent literature cited in the description**

- **MIZOGUCHI I ; OHASHI M ; CHIBA Y ; HASEG-AWA H ; XU M ; OWAKI T ; YOSHIMOTO T.** Prediction of chemical respiratory and contact sensitizers by OX40L expression in dendric cells using a novel 3D coculture system. *Frontiers in Immunology,* 2017, vol. 8, 929
- **LALKO JF ; KIMBER I ; GERBERICK GF ; FOERT-SCH LM ; API AM ; DEARMAN RJ.** The direct peptide reactivity assay: selectivity of chemical respiratory allergens. *Toxicological Sciences,* 2012, vol. 129 (2), 421-431